(19) 

**Europäisches Patentamt**

**European Patent Office**

**Office européen des brevets**

(11) **EP 3 908 822 B1**

(12) **FASCICULE DE BREVET EUROPEEN**

(45) Date de publication et mention de la délivrance du brevet:
**17.07.2024 Bulletin 2024/29**

(21) Numéro de dépôt: **19848790.2**

(22) Date de dépôt: **19.12.2019**

(51) Classification Internationale des Brevets (IPC):
**G01N 3/08** *(2006.01)* **G01N 3/30** *(2006.01)*

(52) Classification Coopérative des Brevets (CPC):
**G01N 3/08; G01N 3/30;** G01N 2203/006;
G01N 2203/0212

(86) Numéro de dépôt international:
**PCT/FR2019/053203**

(87) Numéro de publication internationale:
**WO 2020/144417 (16.07.2020 Gazette 2020/29)**

(54) **PROCEDE DE CONTROLE D'UNE PROPRIETE DE TOLERANCE AUX DOMMAGES D'UNE PIECE REALISEE EN UN ALLIAGE D'ALUMINIUM**

VERFAHREN ZUR ÜBERPRÜFUNG EINER SCHADENSTOLERANZEIGENSCHAFT EINES AUS EINER ALUMINIUMLEGIERUNG GEFERTIGTEN TEILS

METHOD FOR CHECKING A DAMAGE TOLERANCE PROPERTY OF A PART MADE OF AN ALUMINIUM ALLOY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.01.2019 FR 1900193**

(43) Date de publication de la demande:
**17.11.2021 Bulletin 2021/46**

(73) Titulaire: **Constellium Issoire**
**63500 Issoire (FR)**

(72) Inventeurs:
- **LORENZINO, Pablo**
**38500 Voiron (FR)**
- **BAYONA-CARRILLO, Nicolas**
**38500 Coublevie (FR)**

(74) Mandataire: **Constellium - Propriété Industrielle C-TEC Constellium Technology Center Propriété Industrielle Parc Economique Centr'Alp 725, rue Aristide Bergès CS10027 38341 Voreppe (FR)**

(56) Documents cités:
- PARTHEEPAN G ET AL: "Fracture toughness evaluation using miniature specimen test and neural network", COMPUTATIONAL MATERIALS SCIENCE, ELSEVIER, AMSTERDAM, NL, vol. 44, no. 2, 12 June 2008 (2008-06-12), pages 523 - 530, XP025646371, ISSN: 0927-0256, [retrieved on 20080612], DOI: 10.1016/J.COMMATSCI.2008.04.013
- ASTM: "Standard Test Method for Linear-Elastic Plane-Strain Fracture Toughness KIc of Metallic Materials1", TEST METHOD FOR LINEAR-ELASTIC PLANE-STRAIN FRACTURE TOUGHNESS KIC OF METALLIC MATERIALS, 31 December 2013 (2013-12-31), West Conshohocken, PA, XP055631604, Retrieved from the Internet <URL:http://www.astm.org/cgi-bin/resolver.cgi?E399-12E3> [retrieved on 20191014], DOI: 10.1520/E0399-12E03
- ASTM: "StandardTest Method for K-R Curve Determination1", TEST METHOD FOR K-R CURVE DETERMINATION, 31 March 2013 (2013-03-31), West Conshohocken, PA, XP055631606, Retrieved from the Internet <URL:http://www.astm.org/cgi-bin/resolver.cgi?E561-10E2> [retrieved on 20191014], DOI: 10.1520/E0561-10E02

**(Cont. page suivante)**

- DATABASE INSPEC [online] THE INSTITUTION OF ELECTRICAL ENGINEERS, STEVENAGE, GB; 31 December 2006 (2006-12-31), KANG J Y ET AL: "Application of artificial neural network for predicting plain strain fracture toughness using tensile test results", XP002795043, Database accession no. 8953305
- DIN DEUTSCHES INSTITUT FÜR NORMUNG E.V.: "DIN EN 12258-1 Aluminium und Aluminiumlegierungen", 31 December 2012 (2012-12-31), pages 1 - 180, XP055632470, Retrieved from the Internet <URL:https://www.beuth.de/de/norm/din-en-12258-1/151929801> [retrieved on 20191015]
- SWISS MEM: "EN 485-2", 31 December 2016 (2016-12-31), XP055636307, Retrieved from the Internet <URL:https://shop.snv.ch/en/Thematic-Fields/Materials-raw-materials/Metallurgy/Products-of-non-ferrous-metals/Aluminium-products/Aluminium-and-aluminium-alloys-Sheet-strip-and-plate-Part-2-Mechanical-properties-oxid-10.html?cur=1&listtype=search&searchparam=SN%20EN%20485-2> [retrieved on 20191028]
- ABENDROTH M ET AL: "Determination of deformation and failure properties of ductile materials by means of the small punch test and neural networks", COMPUTATIONAL MATERIALS SCIENCE, ELSEVIER, AMSTERDAM, NL, vol. 28, no. 3-4, 31 December 2003 (2003-12-31), pages 633 - 644, XP027136252, ISSN: 0927-0256, [retrieved on 20031027]
- ANASTASIA MULIANA ET AL: "Artificial neural network and finite element modeling of nanoindentation tests", METALLURGICAL AND MATERIALS TRANSACTIONS A, SPRINGER-VERLAG, NEW YORK, vol. 33, no. 7, 31 July 2002 (2002-07-31), pages 1939 - 1947, XP019694108, ISSN: 1543-1940
- A FSC: "INCH-POUND DEPARTMENT OF DEFENSE TEST METHOD STANDARD V 50 BALLISTIC TEST FOR ARMOR", 18 December 1997 (1997-12-18), XP055166893, Retrieved from the Internet <URL:http://www.globalarmour.co.za/downloads/MIL STD 662.pdf> [retrieved on 20150203]
- "NF A50-800-3: Hot-rolled weldable aluminium alloy sheets for armouring - Part 2 : ballistic testing method - Tôles en alliage d'aluminium soudable laminées à chaud pour blindage - Partie 2 : méthode d'essais de tir", 31 August 2014 (2014-08-31), pages 1 - 16, XP009519381, Retrieved from the Internet <URL:https://www.boutique.afnor.org/standard/nf-a50-800-2/hot-rolled-weldable-aluminium-alloy-sheets-for-armouring-part-2-ballistic-testing-method/article/819070/fa177884> [retrieved on 20200317]
- AFNOR: "NF A50-800-3", 31 August 2014 (2014-08-31), XP055677102, Retrieved from the Internet <URL:https://www.boutique.afnor.org/standard/nf-a50-800-3/hot-rolled-weldable-aluminium-alloy-sheets-for-armouring-part-3-characteristics-of-the-ammunitions-and-maximum-protection-veloci/article/819072/fa178461> [retrieved on 20200317]
- NAJIHAH RAHMAN ET AL: "Experimental and Numerical Investigation on the Layering Configuration Effect to the Laminated Aluminium/Steel Panel Subjected to High Speed Impact Test", METALS, vol. 8, no. 9, 17 September 2018 (2018-09-17), pages 732, XP055677204, DOI: 10.3390/met8090732
- KILIC: "Determination of penetration depth at high velocity impact using finiteelement method and artificial neural network tools", 12 January 2015 (2015-01-12), XP055677315, Retrieved from the Internet <URL:https://reader.elsevier.com/reader/sd/pii/S2214914715000021?token=0038D42A0A344774BBF5FEFA6C5D14DD98E948C2E318FEC5442C17FA67171AA50AAC64E4C89C3CE317FCA981A1C92B16> [retrieved on 20200317]

**Description**

## DOMAINE TECHNIQUE

Le domaine technique de l'invention est le contrôle d'une pièce en alliage d'aluminium, et en particulier de pièces destinées à être utilisées en tant qu'élément de structure d'un véhicule ou d'un aéronef.

## ART ANTERIEUR

Les alliages d'aluminium sont couramment utilisés dans l'industrie aéronautique, en particulier pour la fabrication d'éléments de structures ou de voilure. L'aluminium, par sa légèreté, sa tenue à la corrosion et son aptitude à être mis en forme, répond aux attentes de l'aéronautique. Son utilisation dans des alliages permet d'obtenir des matériaux ayant des propriétés mécaniques améliorées. Les alliages les plus couramment utilisés dans l'aéronautique sont les alliages de type 2XXX et 7XXX.

Les exigences des constructeurs aéronautiques portent sur la résistance mécanique ainsi que sur la tolérance aux dommages, par exemple la ténacité, connue sous l'acronyme anglais "Fracture toughness". Le respect de telles exigences impose la réalisation de nombreux essais expérimentaux, en vue de caractériser et quantifier les propriétés mécaniques. L'objectif est de s'assurer que les alliages respectent les spécifications imposées par les constructeurs.

La ténacité, qui représente la résistance à la propagation de fissures, est une propriété importante dans le cas des applications aéronautiques. Des alliages de type AA2050 ou AA2198 présentent par exemple des propriétés de ténacité particulièrement intéressantes. La mesure des propriétés de ténacité est régie par des normes. Par exemple, la norme ASTM E399-12 définit la détermination de la valeur critique du facteur d'intensité de contrainte, usuellement désignée par la notation $K_{IC}$. Cette grandeur caractérise la résistance d'un matériau à la propagation brutale d'une fissure soumise à des contraintes telles que l'état de déformation soit plan. L'acronyme ASTM désigne l'organisme de normalisation "ASTM international".

La grandeur $K_{IC}$ est usuellement déterminée de façon expérimentale, sur une éprouvette préfissurée. L'éprouvette est soumise à une contrainte selon laquelle les surfaces de la fissure se déplacent perpendiculairement au plan de fissure, ce qui correspond à un mode dit "mode par ouverture", connu par l'homme du métier par la désignation "mode I".

D'autre part, la norme ASTM E561-10 définit la définition d'une courbe, dite courbe R, représentant le facteur d'intensité de contrainte effectif en fonction de l'extension de fissue effective. Le facteur d'intensité de contrainte critique Kc, en d'autres termes le facteur d'intensité qui rend la fissure instable, est calculé à partir de la courbe R. Le facteur d'intensité de contrainte $K_{CO}$ est également calculé en attribuant la longueur de fissure initiale au commencement de la charge monotone, à la charge critique. Ces deux valeurs sont calculées pour une éprouvette de la forme requise. $K_{app}$ représente le facteur $K_{CO}$ correspondant à l'éprouvette qui a été utilisée pour effectuer l'essai de courbe R. $K_{eff}$ représente le facteur Kc correspondant à l'éprouvette qui a été utilisée pour effectuer l'essai de courbe R. $\Delta a_{eff(max)}$ représente l'extension de fissure du dernier point valide de la courbe R. La longueur de la courbe R - à savoir l'extension de fissure maximale de la courbe - est un paramètre en lui-même important, notamment pour la conception de fuselage. $K_{R60}$ représente le facteur d'intensité de contrainte effectif pour une extension de fissure effective $\Delta a_{eff}$ de 60 mm.

Cependant, ce type d'essai, expérimental et destructif, est coûteux en temps.

De la même façon, il existe un besoin pour estimer des propriétés de balistiques de produits pour les constructions dans l'armement, en particulier pour les composants de blindage. Les composants de blindage peuvent être utilisés pour la fabrication de parois de coque de blindage et d'appliques complémentaires, qui sont des panneaux amovibles à monter sur les faces externes des véhicules. Le panneau de blindage a une face exposée aux chocs et aux chocs et une face arrière ou de sortie. Lors d'un impact sur un panneau de blindage métallique, le projectile perforant peut-être complètement arrêté dans le panneau, mais les dommages au panneau sur sa face arrière peuvent entraîner la formation de fragments qui sont violemment éjectés du panneau vers l'intérieur du véhicule. L'aptitude à l'impact s'apparente à de la tolérance aux dommages. Les panneaux de blindage sont généralement soumis à deux types de tests. Le premier test, destiné à quantifier leur capacité à arrêter les projectiles perforants, est désigné par les lettres "AP" ("Armor Piercing") et caractérise la résistance à la perforation. Le deuxième test vise à quantifier leur capacité à résister aux impacts qui génèrent des débris fragmentés. Ce deuxième type de test est désigné par l'abréviation "FSP" ("Fragment simulated projectiles"). Lors de ces tests, les panneaux de blindage sont la cible de projectiles de différentes formes et tailles. Pour les deux tests, la capacité d'arrêter les balles et d'absorber leur énergie cinétique est quantifiée par un paramètre appelé vitesse limite de protection (V50).

La détermination de la vitesse limite de protection d'un produit requiert des moyens et des accréditations très spécifiques, qui rendent les essais longs et coûteux. Il existe donc un besoin pour estimer des propriétés de balistiques de produits pour les constructions dans l'armement.

Les alliages les plus couramment utilisés dans le blindage sont des alliages de la série 2XXX, 7XXX, 5XXX et 6XXX.

Certains auteurs ont décrit des procédés d'estimation de propriétés de tolérance aux dommages par des moyens de calcul. La publication Partheepan G. "Fracture toughness evaluation using miniature specimen and neural network", Computational Materials Science 44 (2008) 523-530 décrit le recours à un estimateur mettant en oeuvre un réseau de neurones pour estimer une valeur de ténacité de façon non destructive. Le réseau de neurones a été paramétré à partir d'une phase d'apprentissage, au cours de laquelle ont été établi par modélisation ou par mesures expérimentales des diagrammes mesurant l'allongement en fonction de la charge en utilisant des éprouvettes spéciales dont la valeur de ténacité est connue.

La publication Kang JY "Application of artificial neural network for predicting plain strain fracture toughness using tensile test results", Fatigue Fact Engng Mater 29, 321-329, décrit également le recours à un algorithme basé sur une architecture de type réseaux de neurones pour estimer une propriété de ténacité à partir de propriétés issues d'essais de traction, en particulier la limite d'élasticité, la résistance à la traction, ou l'allongement de rupture.

Les publications précitées exploitent le développement des algorithmes basés sur des architectures de type réseau de neurones. De tels algorithmes sont actuellement accessibles dans des logiciels de calculs couramment utilisés, par exemple l'environnement Matlab ® ou l'environnement Python.

Les inventeurs proposent un procédé de contrôle basé sur une estimation non destructive, d'une valeur de tolérance aux dommages, de façon à limiter le nombre d'essais destructifs réalisés sur des éprouvettes.

## EXPOSE DE L'INVENTION

Un objet de l'invention et un procédé de contrôle d'une propriété de tolérance aux dommages d'une pièce réalisée en alliage d'aluminium, la pièce étant en forme de tôle ou de profilé extrudé, comportant les étapes suivantes :

a) mesurer au moins deux propriétés résultant d'essais de traction de la pièce, dans les directions L(longitudinal) et/ou ST (travers court) et/ou LT (travers long), les propriétés étant choisies parmi :

- la limite d'élasticité ;
- et/ou la résistance à la traction ;
- et/ou l'allongement à la rupture ;

b) prendre en compte une épaisseur de la pièce ;
c) utiliser les propriétés mesurées lors de l'étape a) et l'épaisseur prise en compte lors de l'étape b) en tant que données d'entrée d'un estimateur par réseaux de neurones ;
d) estimer, à l'aide de l'estimateur, la propriété représentative d'une tolérance aux dommages de la pièce;
e) prendre en compte un seuil d'acceptation et un intervalle de confiance, et comparer la propriété estimée lors de l'étape d) au seuil d'acceptation, en tenant compte de l'intervalle de confiance ;
f) en fonction de la comparaison :

- considérer que la pièce satisfait au contrôle ;
- ou considérer que la pièce ne satisfait pas au contrôle.

Par tolérance aux dommages, on entend une propriété caractérisant la résistance à la propagation de fissures. Il s'agit par exemple de la ténacité, cette dernière correspondant à une valeur critique du facteur d'intensité de contrainte. La ténacité est en particulier déterminée selon le protocole défini dans la norme ASTM E399-12. Un autre exemple de tolérance aux dommages peut être associé à la résistance à la perforation, utilisé en particulier pour caractériser les performances de panneaux de blindage. Par exemple, on peut citer la capacité d'arrêter les balles et d'absorber leur énergie cinétique qui est quantifiée par un paramètre appelé vitesse limite de protection (V50) définie selon la NF A50-800 é et 3 (2014).

Par "en tenant compte de l'intervalle de confiance", on entend par exemple que l'intervalle de confiance, éventuellement pondéré d'un facteur de pondération, est soit ajouté au seuil d'acceptation, soit soustrait de la propriété estimée. Cet intervalle de confiance ne correspond pas à un critère de convergence servant à caractériser la validité du modèle dans l'estimateur.

Les termes "tôle" et "profilés" sont définis dans la norme NF EN 12258-1.

Selon un mode de réalisation, lorsque lors de l'étape e), la pièce ne satisfait pas au contrôle, le procédé comporte une étape f) de mesure de la propriété de tolérance aux dommages de la pièce à partir d'une éprouvette prélevée dans ladite pièce.

Le procédé peut comporter l'une des caractéristiques suivantes, prises isolément ou selon les combinaisons techniquement réalisables :

- l'étape c) comporte également une prise en compte d'une concentration d'au moins un élément d'alliage dans l'alliage d'aluminium ;
- l'étape a) comporte une mesure de l'allongement à la rupture et l'étape c) comporte une prise en compte de l'allongement à la rupture ainsi mesurée ;
- l'étape a) comporte une mesure de la limite d'élasticité et l'étape c) comporte une prise en compte de la limite d'élasticité ainsi mesurée ;
- l'étape a) comporte également une mesure d'une propriété de dureté et l'étape c) comporte une prise en compte de la propriété de dureté ainsi mesurée ;
- l'étape a) comporte uniquement une mesure de l'allongement à la rupture et de la limite d'élasticité, l'étape c) comporte une prise en compte de l'allongement à la rupture et de la limite d'élasticité ainsi mesurés ;
- lorsque l'étape d) comporte une estimation de la propriété représentative d'une tolérance aux dommages de la pièce selon les directions L-T (Longitudinal - Travers Long), l'étape c) comporte, au moins, une prise en compte de propriétés mesurées, lors de l'étape a), selon la direction L;
- lorsque l'étape d) comporte une estimation de la propriété représentative d'une tolérance aux dommages de la pièce selon les directions T-L, (Travers Long - Longitudinal), l'étape c) comporte, au moins, une prise en compte de propriétés mesurées, lors de l'étape a), selon la direction LT ;
- lorsque l'étape d) comporte une estimation de la propriété représentative d'une tolérance aux dommages de la pièce selon les directions S-L, (Travers Court - Longitudinal) l'étape c) comporte, au moins, une prise en compte de propriétés mesurées, lors de l'étape a), selon la direction ST.
- l'étape c) comporte une prise en compte de propriétés mesurées, lors de l'étape a), selon différentes directions.
- l'étape c) comporte une prise en compte de trois propriétés mesurées, lors de l'étape a), et cela selon trois directions différentes, en particulier les directions L, ST et LT.
- la propriété tolérance aux dommages est un facteur d'intensité apparent ou une valeur critique d'un facteur d'intensité, également appelé ténacité.
- la propriété de tolérance aux dommages est un facteur d'intensité de contrainte effectif pour une extension de fissure effective prédéterminée, par exemple 60 mm.
- La propriété de tolérance aux dommage est une vitesse limite de protection (V50).
- l'alliage d'aluminium est un alliage de la série 2XXX ou de la série 7XXX, ou de la série 5XXX ou de la série 6XXX.

L'étape c) peut notamment être mise en oeuvre par une unité de traitement, par exemple un microprocesseur.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

[Fig. 1] La figure 1 montre, pour différentes directions, des résultats de mesures de la ténacité en fonction de la limite d'élasticité, pour différents échantillons. Chaque point correspond à un échantillon. Ces résultats concernent des alliages d'aluminium de type AA2050.

[Fig. 2A] La figure 2A schématise une architecture de type réseau de neurones.

[Fig. 2B] La figure 2B représente les principales étapes d'un procédé objet de l'invention.

[Fig. 3A] La figure 3A représente des valeurs de ténacité (valeur critique du facteur d'intensité de contrainte) estimées par un modèle de type réseau de neurones en fonction de valeurs mesurées.

[Fig. 3B] La figure 3B montre un histogramme des erreurs relatives d'estimation de la ténacité.

[Fig. 4A] La figure 4A représente une prise en compte d'un intervalle de confiance, formant une marge de sécurité, dans la définition d'un critère d'acceptation, ce dernier correspondant à une valeur de ténacité.

[Fig. 4B] La figure 4B illustre, pour différentes épaisseurs de pièces, la prise en compte d'un intervalle de confiance, formant une marge de sécurité, par rapport à un critère d'acceptation.

[Fig. 4C] La figure 4C représente une définition d'un niveau de confiance, défini à partir de l'intervalle de confiance, en fonction d'un niveau de risque.

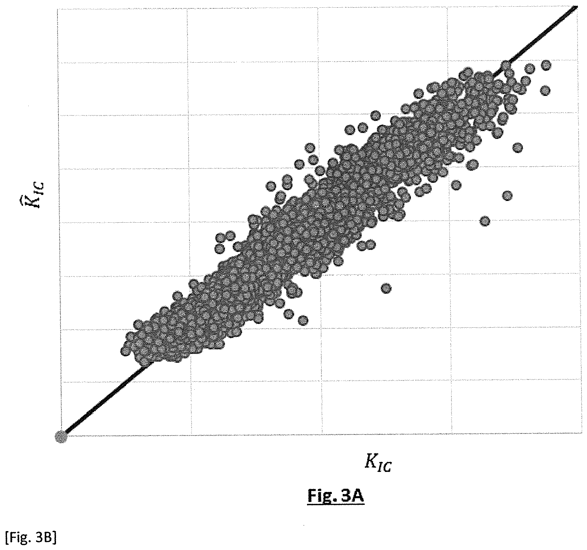

Les figures 3A à 4C ont été établies à partir d'estimations de la ténacité d'alliages d'aluminium de type AA2050.

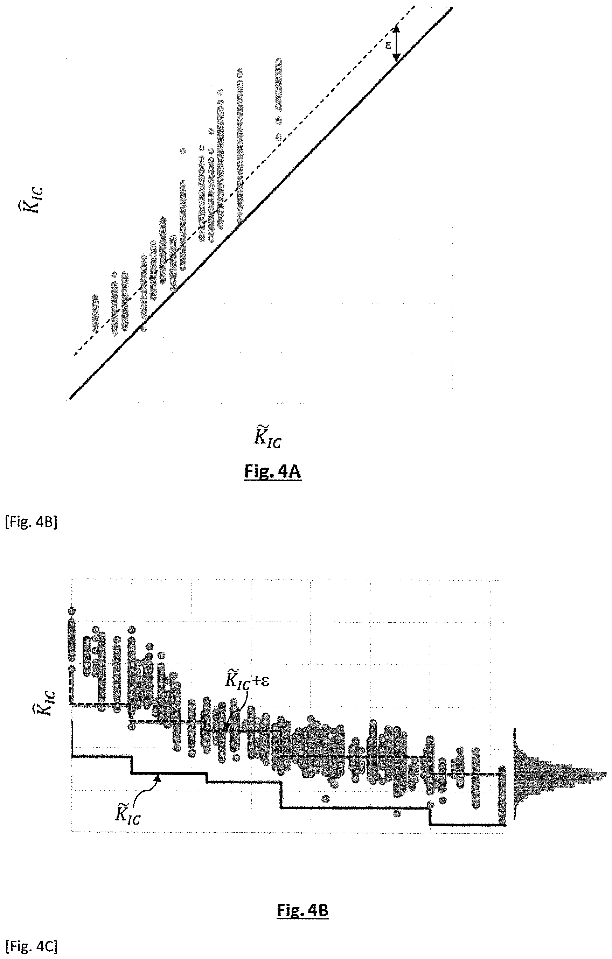

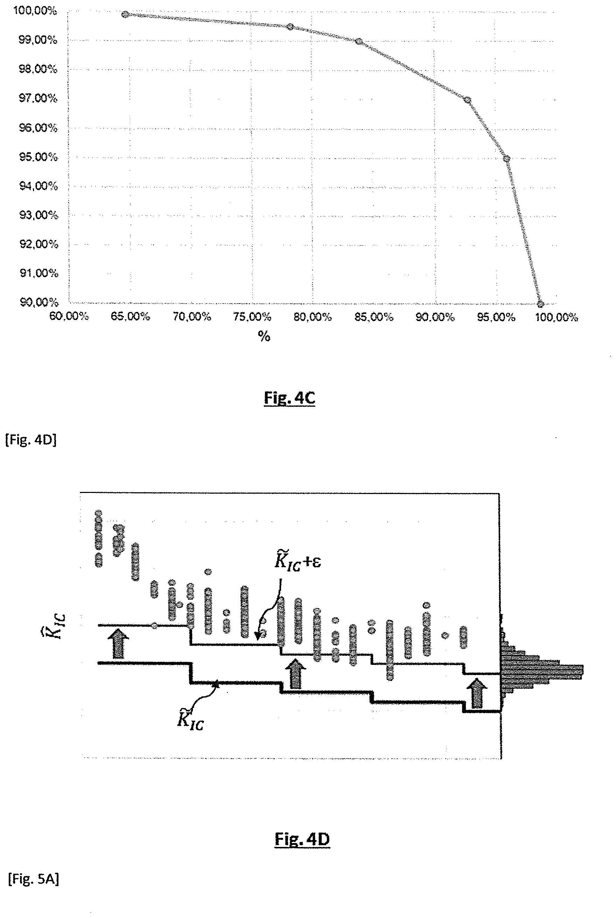

[Fig. 4D] La figure 4D illustre, pour différentes épaisseurs de pièces, la prise en compte d'une marge de sécurité par rapport à une spécification, en utilisant des estimations de la ténacité d'alliages d'aluminium de type AA7050.

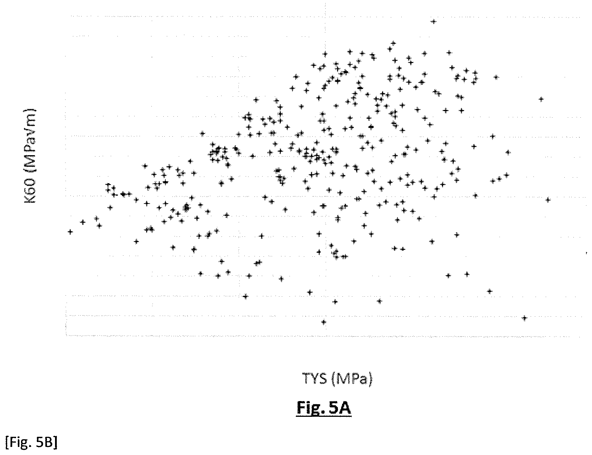

[Fig. 5A] La figure 5A montre des résultats de mesures de tolérance aux dommages en fonction de la limite d'élasticité pour différents échantillons. Sur la figure 5A, la propriété de tolérance aux dommages considérée est un facteur d'intensité de contrainte effectif pour une extension de fissure effective de 60 mm ($K_{R60}$).

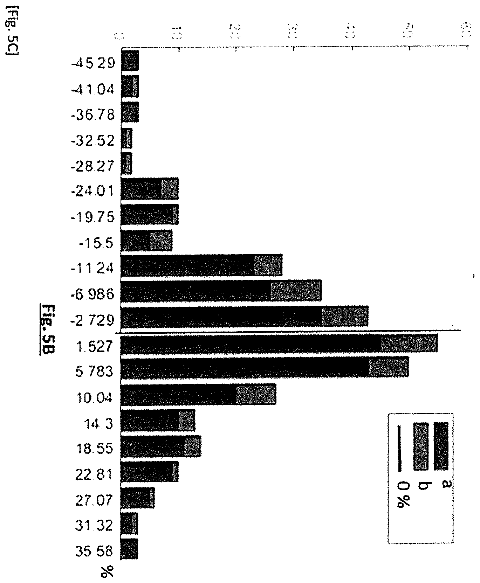

[Fig. 5B] La figure 5B montre un histogramme des erreurs d'estimation d'une propriété de tolérance aux dommages ($K_{app}$) sur différents types d'alliage d'aluminium.

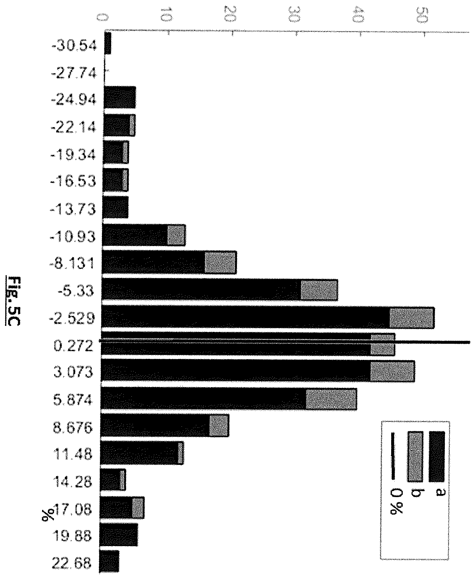

[Fig. 5C] La figure 5C montre un histogramme des erreurs d'estimation d'une autre propriété de tolérance aux dommages ($K_{R60}$) sur différents types d'alliage d'aluminium.

## EXPOSE DE MODES DE REALISATION PARTICULIERS

Le facteur d'intensité de contrainte critique, noté $K_{IC}$, parfois désigné par le terme ténacité, est déterminé selon un protocole d'essai défini dans la norme ASTM E399-12, mentionnée dans l'art antérieur. On soumet une éprouvette préfissurée à une charge croissante. La fissure présente une ouverture, dont on mesure l'évolution en fonction de la charge appliquée à l'éprouvette. Une courbe, représentant la charge appliquée en fonction de l'ouverture, est obtenue, en fonction de laquelle un facteur d'intensité de contrainte $K_Q$ est déterminé, ce dernier correspondant à une intersection de la courbe précitée et d'une droite de pente prédéterminée. Dans certaines conditions, précisées dans le paragraphe 9 de la norme précitée, le facteur d'intensité de contrainte $K_Q$ correspond à une mesure valide du facteur d'intensité de contrainte critique $K_{IC}$. Lorsque ces conditions sont réunies, on considère que le facteur d'intensité de contrainte critique $K_{IC}$ caractérise le matériau, en étant indépendant de la géométrie de l'éprouvette considérée. Cette grandeur qui correspond à la ténacité en déformation plane est également ici désignée simplement par le terme "ténacité".

Le facteur d'intensité de contrainte apparent à la rupture $K_{app}$, qui correspond à la ténacité en contrainte plane, est obtenu en établissant une courbe désignée par le terme "courbe R", selon un protocole d'essai défini dans la norme ASTM E561. La courbe R représente une évolution du facteur d'intensité de contrainte critique $K_C$ pour la propagation de fissure, en fonction de la longueur de fissure, sous une contrainte monotone et croissante. La courbe R permet une détermination de la charge critique pour une rupture instable. On peut également déterminer un facteur d'intensité de contrainte $K_{CO}$, en attribuant une longueur de fissure initiale, avant l'application de la charge. Le facteur d'intensité de contrainte apparent à la rupture $K_{app}$ correspond au facteur $K_{CO}$ correspondant à l'éprouvette qui a été utilisée pour établir la courbe R. Le coefficient $K_{R60}$ est le facteur d'intensité de contrainte effectif pour une extension de fissure effective de 60 mm.

La vitesse limite de protection est définie par exemple dans la norme NF A 50-800-2 et 3 (2014) ou MIL-STD-662 (1997). C'est la vitesse à laquelle la probabilité de pénétration d'un blindage est de 50%. La vitesse limite de protection est la moyenne d'un nombre pair de vitesses à l'impact, au moins égal à 4, dont une moitié correspond à des protections, et la seconde moitié à des non protections. Elle est établie en calculant la moyenne des vitesses atteintes par les projectiles à l'impact résultant de la prise du même nombre de résultats ayant les vitesses les plus élevées correspondant à une pénétration partielle et les résultats ayant les vitesses les plus faibles correspondant à une pénétration complète. Une pénétration complète se produit lorsque le projectile impactant ou tout fragment (du projectile ou de l'éprouvette) perce une fine plaque témoin située derrière l'éprouvette.

Elément structurel : un élément structurel d'une construction mécanique est une pièce pour laquelle les propriétés mécaniques statiques et/ou dynamiques sont particulièrement importantes pour l'intégrité de la structure. Dans une construction aéronautique, il s'agit notamment, et de façon non limitative, des éléments composant le fuselage, les ailes, l'empennage, la dérive.

Relativement aux essais de traction, les termes sens travers, sens long (L), sens travers-long (TL), sens travers-court (TC) sont définis dans la norme NF EN 485. Ils correspondent respectivement aux désignations anglosaxonnes Longitudinal (L), Long Transverse (LT ou T), Short Transverse (ST ou S). Dans la suite du texte, on se référera aux acronymes anglosaxons L, LT et ST.

Pour les essais relatifs à la détermination de la tenue aux dommages, les directions L-T, T-L et S-L sont définies dans la norme ASTM E399-12, paragraphes 3.1.3.2 et 3.1.3.4. La première lettre correspond à une direction normale au plan de fissure. La deuxième lettre correspond à une direction de propagation de la fissure. Dans ces désignations, on se réfère à la nomenclature anglosaxonne : L = longitudinal ; T = Long Transverse ; S = Short Transverse.

L'invention s'applique à des alliages d'aluminium, et en particulier à des alliages d'aluminium des séries 2XXX, 7XXX ou 5XXX. La désignation des alliages est effectuée selon la nomenclature définie par The American Aluminum Association. L'invention permet le contrôle d'une pièce réalisée en alliage d'aluminium, et plus précisément le contrôle d'une propriété de tolérance aux dommages de la pièce. La pièce peut notamment être une tôle.

L'invention tire profit d'un nombre très important de pièces d'alliages d'aluminium ayant subi des caractérisations mécaniques ou chimiques précises, parmi lesquelles :

- la composition chimique ;
- l'épaisseur ;
- des propriétés mécaniques de traction, en particulier la limite d'élasticité (ou Tensile Yield Strength), la résistance à la traction (Ultimate Tensile Strength), l'allongement relatif, ou allongement à la rupture ;
- des propriétés représentatives de la tolérance aux dommages, par exemple la ténacité ($K_{IC}$), le facteur d'intensité apparent à la rupture (K$_{app}$) ou le facteur d'intensité K$_{R60}$ ou la vitesse limite de pénétration (V50) préalablement défini.

Les inventeurs ont eu par exemple accès à des données de tolérance aux dommages relatives à 6200 pièces d'alliage de type AA2050, et cela selon les directions L-T, T-L et S-T. Cela représente des données de caractérisation très importantes. Ils disposaient également de données d'essais de résistance à la traction selon les directions L, LT et ST.

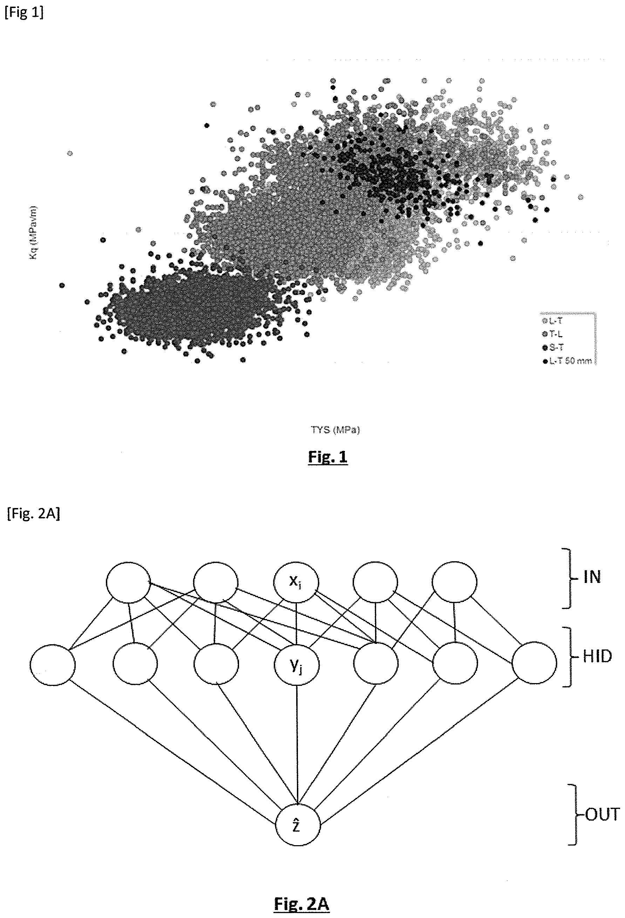

On a représenté, sur la figure 1, un nuage de points, chaque point représentant un échantillon prélevé sur une pièce. Pour chaque échantillon, on a représenté la ténacité (K$_{IC}$ - axe des ordonnées) en fonction de la limite d'élasticité (axe des abscisses). Le niveau de gris de chaque point correspond à une direction telle que précédemment définie, la légende étant la suivante :

- L-T : ténacité selon L-T, limite d'élasticité selon L ;
- T-L : ténacité selon T-L, limite d'élasticité selon LT ;
- S-T : ténacité selon S-T, limite d'élasticité selon ST ;
- L-T 50mm : ténacité selon L-T, limite d'élasticité selon L-épaisseur des tôles égale à 50 mm.

On observe une certaine corrélation entre la limite d'élasticité et la ténacité.

Les inventeurs ont développé un algorithme d'estimation de propriétés caractérisant la tolérance aux dommages, en fonction de données d'entrée représentatives de la résistance à la traction. Pour cela, on a utilisé une partie des résultats de caractérisation disponibles pour former un ensemble d'apprentissage, permettant un paramétrage de l'algorithme. Une autre partie des résultats de caractérisation disponibles ont été utilisés pour former un ensemble de test de l'algorithme après son paramétrage. 80% des données disponibles ont été utilisées pour former l'ensemble d'apprentissage. 20% des données disponibles ont été utilisées pour former l'ensemble de test.

L'algorithme utilisé est un réseau de neurones comportant une couche d'entrée, comportant les données d'entrée x$_i$, une couche intermédiaire, ou couche cachée, et une couche de sortie, formant la grandeur à estimer, en l'occurrence une propriété de tolérance aux dommages, par exemple la ténacité. La couche intermédiaire forme une couche cachée, comportant des noeuds y$_j$, ou neurones. A chaque noeud y$_j$, et pour chaque donnée d'entrée x$_i$, correspond un facteur de pondération w$_{i,j}$ déterminé au cours de la phase d'apprentissage. Les inventeurs ont programmé l'algorithme sous l'environnement MATLAB ®, logiciel fourni par la société The Mathworks, en mettant en oeuvre le module "ANN Toolbox". L'apprentissage permet de déterminer, entre autres, les facteurs de pondération de la couche cachée. Dans l'exemple considéré, la couche cachée comporte 30 noeuds. Chaque noeud est relié à une donnée d'entrée par un facteur de pondération et un biais.

La figure 2A schématise l'architecture d'un réseau de neurones tel que celui mis en oeuvre par les inventeurs. Le réseau comporte 3 couches :

- la couche d'entrée IN, comportant les variables d'entrée x$_i$. l'indice i est un entier compris entre 1 et N$_i$, N$_i$ étant un entier supérieur ou égal à 2. Chaque variable d'entrée est :

    soit une propriété mécanique de traction, en particulier l'allongement relatif et/ou la limite d'élasticité et/ou la résistance à la traction ;
    soit l'épaisseur de la pièce ;
    soit une fraction massique d'un élément d'alliage, par exemple une fraction massique de Cu ou une fraction massique de Li.

- la couche cachée HI, comportant des noeuds (ou neurones) y$_j$, usuellement désignés par le terme anglosaxon "node". L'indice j est un entier compris entre 1 et N$_j$, N$_j$ étant un entier supérieur ou égal à 5 ou 10. Dans cet exemple N$_j$ = 30.
- la couche de sortie OUT, comportant une variable de sortie $\hat{z}$. Dans notre exemple, la couche de sortie ne comporte qu'une unique variable de sortie $\hat{z}$, qui correspond à la propriété de tolérance aux dommages estimée par l'algorithme. Il peut s'agir d'une estimation de la ténacité en déformation plane ($\hat{K}_{IC}$), mais également d'autres paramètres, par exemple la ténacité en contrainte plane $\hat{K}_{app}$ ou $\hat{K}_{R60}$.

Chaque noeud de la couche intermédiaire est relié à chaque donnée d'entrée. Sur la figure 2A, on n'a pas représenté toutes les liaisons, à des fins de clarté.

L'algorithme est mis en oeuvre par une unité de traitement de données, par exemple un microprocesseur, reliée à une mémoire comportant l'algorithme et son paramétrage. L'algorithme utilise des données physiques mesurées, correspondant aux données d'entrée x$_i$ mentionnées ci-dessus.

A chaque noeud y$_j$ est attribué un facteur de pondération w$_{i,j}$ associé à une variable d'entrée x$_i$. Ainsi, chaque facteur de pondération w$_{i,j}$ est associé à une donnée d'entrée x$_i$ et à un noeud y$_j$. A chaque noeud est également une valeur de biais $w_{0,j}$. Les facteurs de pondération $w_{i,j}$ ainsi que le biais $w_{0,j}$ de chaque noeud sont déterminés au cours de la phase d'apprentissage. Chaque noeud $y_j$ met en oeuvre une fonction d'activation $f_j$, de telle sorte que :
[Math]

$$y_j = f_j \left( w_{0,j} + \sum_i w_{i,j}\, x_i \right) \quad (1)$$

Dans l'architecture mise en oeuvre par les inventeurs, chaque fonction d'activation $f_j$ est une fonction de type tangente hyperbolique. Les valeurs de chaque noeud $y_j$ sont combinées pour former la valeur de la variable de sortie $\hat{z}$.

L'algorithme ayant été paramétré par l'ensemble d'apprentissage, on a effectué des tests visant à évaluer la précision de l'algorithme, c'est-à-dire l'écart entre la ténacité mesurée et la ténacité estimée. La figure 3A montre une courbe dont chaque point correspond à un échantillon de test. Les axes des abscisses et des ordonnées correspondent respectivement à des valeurs de ténacité ($K_{IC}$) mesurées et estimées par l'algorithme. On observe que la dispersion autour de la droite d'équation $y = x$ est faible. La courbe de la figure 3A a été effectuée en considérant des échantillons dans toutes les directions et de différentes épaisseurs, les épaisseurs étant comprises entre 30 et 200 mm.

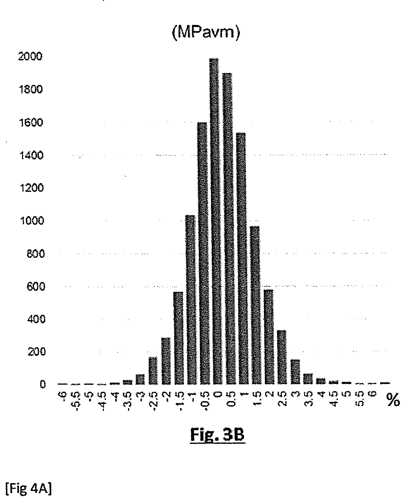

La figure 3B correspond à un histogramme des erreurs relatives (en °/) entre les valeurs de ténacité respectivement mesurées et estimées par l'algorithme. Cet histogramme permet d'évaluer une incertitude d'estimation de l'algorithme. L'écart type $\sigma_{KIC}$ de cet histogramme est estimé à 1.3 MPa√m, ce qui permet de quantifier l'incertitude d'estimation.

La figure 4A est un graphe, représentant en abscisse, une valeur exigée de ténacité, cette valeur étant par exemple issue d'une spécification d'un constructeur. En ordonnée, on a représenté des valeurs de ténacité estimées par l'algorithme. La ligne en trait plein correspond à la droite d'équation $y = x$. On a tracé une ligne en pointillés dont l'écart, par rapport à la droite $y = x$, correspond à une prise en compte d'une marge d'erreur, formant un intervalle de confiance. Selon une définition usuelle, l'intervalle de confiance correspond à n fois l'écart type $\sigma_{KIC}$, où n est un réel positif. *n* peut être par exemple égal à 2.

Ce graphe illustre comment l'incertitude liée à l'estimation de la ténacité peut être prise en compte pour s'assurer du respect d'une exigence issue d'une spécification, n x $\sigma_{KIC}$ correspond à l'intervalle de confiance que l'on prend en compte, de façon à tenir compte de l'incertitude liée à l'estimation. Ainsi, si $\tilde{K}_{IC}$ correspond à un seuil d'acceptation de la ténacité, défini dans une spécification, et si $\hat{K}_{IC}$ correspond à l'estimation de la ténacité issue de l'algorithme, le respect de la spécification peut être tel que :

- si
[Math]

$$\tilde{K}_{IC} + n \times \sigma_{KIC} \leq \hat{K}_{IC} \quad (2)$$

alors l'échantillon testé est considéré comme conforme au seuil d'acceptation défini dans la spécification : la pièce satisfait ainsi au contrôle.

- Si
[Math]

$$\tilde{K}_{IC} + n \times \sigma_{KIC} > \hat{K}_{IC} \quad (3)$$

alors l'échantillon testé est considéré comme non conforme au seuil d'acceptation défini dans la spécification : sur la base de l'estimation $\hat{K}_{IC}$, la pièce ne satisfait pas au contrôle.

Ainsi, d'une façon générale, on peut évaluer, à partir de l'ensemble de test, un indicateur statistique $\sigma_{KIC}$ représentatif de la dispersion des estimations de ténacité $\hat{K}_{IC}$ par rapport aux valeurs exactes de la ténacité. L'indicateur statistique permet de définir un intervalle de confiance $\varepsilon = n \times \sigma_{KIC}$ qui est :

- soit additionné au seuil d'acceptation $\tilde{K}_{IC}$, auquel cas on compare la valeur $\tilde{K}_{IC} + \varepsilon$ à l'estimation $\hat{K}_{IC}$.
- soit retranché de l'estimation $\hat{K}_{IC}$, auquel cas on compare la valeur $\hat{K}_{IC} - \varepsilon$ au seuil d'acceptation $\tilde{K}_{IC}$.

La figure 3B montre une généralisation de ce procédé sur des échantillons de différentes épaisseurs *Th,* à chaque épaisseur correspondant une spécification de ténacité $\tilde{K}_{IC,Th}$. Chaque valeur seuil $\tilde{K}_{IC,Th}$ est augmentée de ε. Les échantillons dont la ténacité estimée est inférieure à $\hat{K}_{IC}$ + ε sont considérés comme non conformes. L'intervalle de confiance peut dépendre de l'épaisseur.

Les échantillons considérés comme non conformes peuvent faire l'objet d'une détermination expérimentale de leur ténacité, de façon à s'assurer de leur conformité ou de leur non-conformité par rapport à la spécification définissant le seuil d'acceptation. La mesure expérimentale est effectuée en prélevant une éprouvette sur la pièce contrôlée. La valeur de ténacité issue de la mesure expérimentale est ensuite à nouveau comparée au seuil d'acceptation $\tilde{K}_{IC}$.

On comprend que le procédé permet d'éviter d'effectuer une mesure expérimentale de la ténacité pour l'ensemble des pièces telles que :

[Math]

$$\tilde{K}_{IC} + \varepsilon \leq \hat{K}_{IC} \quad (4)$$

Plus la valeur de n augmente, plus le pourcentage de tests évités diminue. La figure 4C représente le pourcentage de tests évités en fonction d'un niveau de confiance associé à l'intervalle de confiance ε, ce dernier correspondant à $n \times \sigma_{KIC}$. Un niveau de confiance de 100% conduit à effectuer une mesure expérimentale sur tous les échantillons.

Les figures 3A, 3B, 4A à 4C ont été réalisées en considérant un alliage d'aluminium de type AA2050. La figure 4D est analogue à la figure 4C, en considérant un alliage d'aluminium de type AA7050.

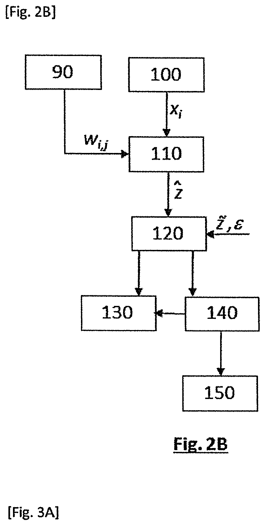

La figure 2B résume les principales étapes d'un procédé de contrôle d'une pièce selon l'invention.

Etape 100 : détermination des données d'entrée $x_i$ de l'algorithme. Tout ou partie des données d'entrée sont déterminées de façon expérimentale.

Etape 110 : mise en oeuvre de l'algorithme, de façon à obtenir une valeur de la variable de sortie $\hat{z}$ correspondant à une estimation de la propriété de tolérance aux dommages considérée. Dans les exemples donnés en lien avec les figures 3A, 3B, 4A à 4C, $\hat{z} = \hat{K}_{IC}$

Etape 120 : prise en compte d'un intervalle de confiance ε et d'un seuil d'acceptation $\tilde{z}$. Comparaison la propriété estimée $\hat{z}$ au seuil d'acceptation $\tilde{z}$ en tenant compte de l'intervalle de confiance ε. Ce dernier est soit soustrait de la propriété estimée $\hat{z}$ soit ajouté au seuil d'acceptation $\tilde{z}$.

En fonction de la comparaison :

- Etape 130 : acceptation de la pièce contrôlée ; ou
- Etape 140 : détermination expérimentale de la propriété de tolérance aux dommages z, en fonction de quoi la pièce est soit acceptée (étape 130), soit rejetée (étape 150).

L'algorithme utilisé dans l'étape 110 a préalablement fait l'objet d'un apprentissage, en utilisant des échantillons d'apprentissage. L'apprentissage fait l'objet d'une étape 90, au cours de laquelle on définit le nombre de couches cachées, le nombre de noeuds par couche cachée ainsi que les fonctions d'activation associées aux noeuds de la couche cachée. L'apprentissage comporte une optimisation, permettant de définir les facteurs de pondération $w_{i,j}$ pour chaque couple donnée d'entrée $x_i$ - noeud $y_j$ ainsi que le biais $w_{0,j}$ associé à chaque noeud.

Les inventeurs ont pu pour des tôles en alliage AA2050 estimer la puissance prédictive des différentes variables d'entrée $x_i$ considérées. Le tableau 1 montre, pour chaque donnée d'entrée, la puissance prédictive. Il s'agit d'un réel compris entre 0 et 1, quantifiant l'importance relative de chaque donnée d'entrée dans l'estimation du résultat.

[Tableau 1]

Tableau 1

| Variable d'entrée $x_i$ | Puissance prédictive |
|---|---|
| Allongement à la rupture | 0,88 |
| Limite d'élasticité | 0,57 |
| Epaisseur | 0,36 |
| Résistance à la Traction | 0,16 |
| Li (%) | 0,08 |

(suite)

| Variable d'entrée $x_i$ | Puissance prédictive |
|---|---|
| Cu (%) | 0,07 |

Les variables d'entrée ayant le plus d'influence sur l'estimation de la ténacité sont donc l'allongement à la rupture, la limite d'élasticité et l'épaisseur. Les fractions massiques de lithium et de cuivre n'ont qu'une importance presque négligeable dans la détermination de la fraction massique.

Les inventeurs ont développé un estimateur, similaire à celui décrit précédemment, qui permet d'estimer des facteurs d'intensité tels le facteur d'intensité de contrainte apparente $K_{app}$ ou le facteur d'intensité $K_{R60}$ préalablement défini. La figure 5A représente une variation de la limite d'élasticité (axe des abscisses) en fonction du facteur d'intensité $K_{R60}$. Chaque croix correspond à un échantillon. De même que sur la figure 1, on observe une certaine corrélation entre la valeur de la limite d'élasticité et la valeur du facteur d'intensité $K_{R60}$.

Les figures 5B et 5C représentent un histogramme des erreurs relatives (en °/) d'estimations relatives du facteur d'intensité de contrainte apparente et du facteur d'intensité $K_{R60}$. Ces histogrammes ont été élaborés à partir de différents types d'alliage, ce qui explique une plus grande dispersion des valeurs d'erreurs relatives représentées sur la figure 1B. Sur chacune de ces figures, les niveaux de gris foncés (a) correspondent à des valeurs d'erreurs relatives obtenues à l'aide d'échantillons d'apprentissage, tandis que les niveaux de gris moins foncés (b) correspondent à des valeurs d'erreurs relatives obtenues à l'aide d'échantillons de test. On a également représenté une droite correspondant à une erreur relative nulle.

Selon un mode de réalisation, les données d'entrée de l'algorithme d'estimation peuvent comporter des valeurs de dureté mesurées sur la pièce contrôlée.

On a effectué d'autres essais, de façon à évaluer l'influence des données d'entrée sur la précision de l'estimation de la ténacité $\hat{K}_{IC}$. Dans ces essais, on a séparé les estimations de la ténacité $\hat{K}_{IC}$ dans les directions respectives L-T, T-L et S-L. On a également pris en compte les directions considérées lors de la réalisation des essais de traction.

Dans une série d'essais, les produits analysés étaient des tôles d'alliage AA2050 T851. Les paramètres de ces essais sont représentés dans le tableau 2A. Les résultats de ces essais ont listés dans le tableau 2B.

Dans le tableau 2A, les colonnes correspondent respectivement aux données suivantes :

- première colonne "Ref" : référence de l'essai ;
- deuxième colonne "Nb données" : nombre d'échantillons considérés. 80% des échantillons forment l'ensemble l'apprentissage du modèle. 20% des échantillons servent à forment l'ensemble de test;
- troisième colonne "Epaisseur" : prise en compte (X) ou non prise en compte de l'épaisseur dans le modèle ;
- quatrième colonne "Elarg" : prise en compte (X) ou non prise en compte un facteur d'élargissement quand un élargissement est effectué pendant le laminage;
- cinquième colonne "Orient" : prise en compte de l'orientation, comme détaillé en lien avec les essais 7 et 8
- sixième, septième et huitième colonne : prise en compte (X) ou non prise en compte de propriétés résultant d'essais de traction (respectivement UTS, TYS et A%) dans la direction L.
- neuvième, dixième et onzième colonne : prise en compte (X) ou non prise en compte de propriétés résultant d'essais de traction (respectivement UTS, TYS et A%) dans la direction LT.
- douzième colonne, treizième colonne et quatorzième colonne : prise en compte (X) ou non prise en compte de propriétés résultant d'essais de traction (respectivement UTS, TYS et A°/) dans la direction TS.

Dans le tableau 2B, les colonnes correspondent respectivement aux données suivantes:

- première colonne "Ref" : référence de l'essai ;
- deuxième, troisième et quatrième colonne : estimation (X) ou non estimation de la ténacité respectivement dans les directions L-T, T-L et S-L ;
- cinquième colonne : écart type de l'erreur d'estimation ;
- sixième colonne : écart type de l'erreur relative d'estimation ;
- septième colonne : erreur relative moyenne d'estimation.

Les données relatives aux erreurs d'estimation, figurant dans les cinquièmes, sixièmes et septièmes colonnes, sont obtenues suite à l'estimation de la ténacité avec chaque ensemble de test.

Dans les essais 1, 2 et 3, on a élaboré et utilisé trois estimateurs, chaque estimateur étant respectivement dédié à l'estimation de la ténacité selon les directions L-T, T-L et S-L, à partir de données de résistance à la traction respectivement mesurées selon les directions L, LT et ST. Chaque estimateur prenait en compte l'épaisseur de la pièce.

Lors de l'essai 1, l'estimateur était configuré en utilisant des données de résistance à la traction mesurées selon la direction L. L'estimateur ainsi paramétré a permis d'estimer la ténacité selon les directions L-T. Lors de l'essai 2, l'estimateur était configuré en utilisant des données de résistance à la traction mesurées selon la direction LT. L'estimateur ainsi paramétré a permis d'estimer la ténacité selon les directions T-L. Lors de l'essai 3, l'estimateur était configuré en utilisant des données de résistance à la traction mesurées selon la direction ST. L'estimateur ainsi paramétré a permis d'estimer la ténacité selon les directions S-L. Les estimations sont correctes, avec une erreur moyenne inférieure à 4%.

Dans les essais 4, 5 et 6, on a élaboré et utilisé trois estimateurs, permettant respectivement d'estimer la ténacité selon les trois directions L-T, T-L et S-L, à partir de données de résistance à la traction mesurées dans les trois directions L, LT et ST. Les estimateurs prenaient également en compte l'épaisseur de la pièce.

On observe que les essais 4, 5 et 6 aboutissent à des erreurs d'estimation réduites par rapport aux essais 1, 2 et 3. Cela montre que pour estimer une ténacité selon une direction déterminée (par exemple la direction L-T), il est préférable de disposer de données d'entrée, résultant d'essais de traction, non pas dans une seule direction (en l'occurrence L pour la direction L-T), mais selon trois directions (L, LT et ST). Ainsi, la prise en compte de données relatives à la résistance à la traction mesurées dans de multiples directions, par exemple deux ou trois directions différentes, permet de réduire l'erreur d'estimation de la ténacité, et cela quelle que soit les directions considérées pour l'estimation de la ténacité.

Dans les essais 7 et 8, on a utilisé un même estimateur, élaboré avec des données d'essais en traction selon trois directions (L, LT et ST). Au cours de l'essai 8, on a pris en compte l'épaisseur de la pièce, ainsi que le facteur d'élargissement après laminage. Au cours de l'essai 7, l'épaisseur de la pièce n'a pas été prise en compte. Lors de la mise en oeuvre de l'estimateur, l'opérateur a sélectionné les directions (L-T, T-L ou S-L), ce qui correspond à la colonne 6 du tableau :

- lorsque la direction sélectionnée est L-T, les données d'entrée sont les propriétés de résistance à la traction mesurées dans la direction L;
- lorsque la direction sélectionnée est T-L, les données d'entrée sont les propriétés de résistance à la traction mesurées dans la direction LT ;
- lorsque la direction sélectionnée est S-L, les données d'entrée sont les propriétés de résistance à la traction mesurées dans la direction ST.

On voit que la prise en compte de l'épaisseur de la pièce permet de réduire l'erreur de d'estimation.

Il résulte de ce qui précède qu'il est optimal de prendre en compte l'épaisseur de la pièce contrôlée, ainsi que des propriétés de résistance à la traction selon différentes directions. Cela réduit l'erreur d'estimation et augmente la répétabilité des mesures.

Dans une série d'essais, les produits analysés étaient des tôles d'alliage AA7050 T7451. Les paramètres de ces essais sont représentés dans le tableau 3A. Les résultats de ces essais ont listés dans le tableau 3B. Les colonnes des tableaux 3A et 3B comportent les mêmes données que les colonnes des tableaux 2A et 2B respectivement.

Les essais 9, 10 et 11 sont similaires aux essais 1,2,3 préalablement décrits en lien avec les tableaux 2A et 2B.

L'essai 12 est similaire à l'essai 8, le facteur d'élargissement n'ayant pas été prise en compte.

L'essai 12 sert de référence de comparaison aux essais 13 à 17. Dans les essais 13 à 17, l'estimateur a été paramétré et utilisé sans prendre en compte au moins un des paramètres considérés lors de l'essai 12 :

- lors de l'essai 13, l'épaisseur de la pièce n'a pas été prise en compte ;
- lors de l'essai 14, l'allongement à la rupture n'a pas été pris en compte ;
- lors de l'essai 15, la résistance à la traction n'a pas été prise en compte ;
- lors de l'essai 16, la limite d'élasticité n'a pas été prise en compte ;
- lors de l'essai 17, la résistance à la traction et la limite d'élasticité n'ont pas été prises en compte.

Il résulte des essais 12 à 17 que la non prise en compte de propriétés de résistance à la traction augmente l'erreur d'estimation. Il semble particulièrement optimal de considérer les trois propriétés mécaniques traction (résistance à la traction, limite d'élasticité, allongement à la rupture), ainsi que l'épaisseur de la pièce. De plus, les propriétés mécaniques de traction doivent être déterminées selon une direction propice aux directions considérées pour estimer la ténacité : en l'occurrence L, LT et ST pour estimer la ténacité dans les directions L-T, T-L et S-T respectivement.

L'invention permet d'éviter un recours systématique à des essais destructifs pratiqués sur des éprouvettes, ces derniers étant réservés sur les pièces dont la valeur de tolérance aux dommages estimée n'est pas suffisamment éloignée du seuil d'acceptation, moyennant un niveau de confiance prédéterminé. Elle ouvre la voie à une nouvelle approche dans le contrôle de pièces destinées à des applications à haut niveau d'exigence, par exemple dans le domaine des véhicules ou des aéronefs.

Dans une autre série d'essai n°18 (voir Tableau 4) , le produit analysé était des tôles d'alliage AA 5083 H131 pour lesquelles la vitesse limite de pénétration (V50) a été estimée en tenant compte des caractéristiques en traction dans le sens TL à mi-épaisseur de produit, c'est-à-dire la limite d'élasticité, la résistance en traction et l'allongement mesurée à mi-épaisseur dans le sens TL. Les résultats obtenus sont listés dans le Tableau 4. Dans le tableau 4, les colonnes correspondent respectivement aux données suivantes :

- première colonne "Ref" : référence de l'essai ;
- deuxième colonne : nombre de données prises en compte, ici 448,
- troisième, quatrième, cinquième et sixième colonne : paramètres pris en compte (X) ;
- septième colonne : type de valeurs estimées, ici la vitesse limite de protection (V50) ;
- huitième colonne : écart type de l'erreur d'estimation ;
- Neuvième colonne : écart type de l'erreur relative d'estimation ;
- Dixième colonne : erreur relative moyenne d'estimation.

Il est ainsi montré que la démarche préalablement décrite pour la ténacité s'applique à la vitesse limite de pénétration (V50). Il est d'ailleurs remarquable que l'écart type de l'erreur relative est faible, indiquant une excellente prédiction par la méthode.

[Tableau 2A]

Tableau 2A

| Ref | Nbdonnées | Epaisseur | Elarg. | Orient. | L UTS | L TYS | L A% | LT UTS | LT TYS | LT A% | TSUTS | TSTYS | TS A% |
|---|---|---|---|---|---|---|---|---|---|---|---|---|---|
| 1 | 6435 | X | | | X | X | X | | | | | | |
| 2 | 6434 | X | | | | | | X | X | X | | | |
| 3 | 5544 | X | | | | | | | | | X | X | X |
| 4 | 6433 | X | | | X | X | X | X | X | X | X | X | X |
| 5 | 6432 | X | | | X | X | X | X | X | X | X | X | X |
| 6 | 5543 | X | | | X | X | X | X | X | X | X | X | X |
| 7 | 18321 | | | X | X | X | X | | | | | | |
|  |  | | | X | | | | X | X | X | | | |
|  |  | | | X | | | | | | | X | X | X |
| 8 | 18321 | X | X | X | X | X | X | | | | | | |
|  |  | | | X | | | | X | X | X | | | |
|  |  | | | X | | | | | | | X | X | X |

13

[Tableau 2B]

Tableau 2B

| Ref | L-T KIC/Kq | T-L KIC/Kq | S-L KIC/Kq | σ(ε) | σ(ε) (%) | Mean(ε) (%) |
|---|---|---|---|---|---|---|
| 1 | X | | | 1.64 | 4.65 | 3.60 |
| 2 | | X | | 1.02 | 3.46 | 2.69 |
| 3 | | | X | 1.31 | 5.17 | 4.02 |
| 4 | X | | | 1.42 | 4.03 | 3.07 |
| 5 | | X | | 0.91 | 3.12 | 2.40 |
| 6 | | | X | 1.24 | 4.91 | 3.80 |
| 7 | X | | | 1.65 | 5.31 | 4.07 |
| | | X | | | | |
| | | | X | | | |
| 8 | X | | | 1.26 | 4.19 | 3.17 |
| | | X | | | | |
| | | | X | | | |

[Tableau 3A]

Tableau 3A

| Ref | Nb données | Epaisseur | Elarg. | Orient. | L UTS | L TYS | L A% | LT UTS | LT TYS | LT A% | TS UTS | TS TYS | TS A% |
|---|---|---|---|---|---|---|---|---|---|---|---|---|---|
| 9 | 1480 | X | | | X | X | X | | | | | | |
| 10 | 1480 | X | | | | | | X | X | X | | | |
| 11 | 1480 | X | | | | | | | | | X | X | X |
| 12 | 4440 | X | | X | X | X | X | | | | | | |
| | | | | X | | | | X | X | X | | | |
| | | | | X | | | | | | | X | X | X |
| 13 | 4440 | | | X | X | X | X | | | | | | |
| | | | | X | | | | X | X | X | | | |
| | | | | X | | | | | | | X | X | X |
| 14 | 4440 | X | | X | X | X | | | | | | | |
| | | | | X | | | | X | X | | | | |
| | | | | X | | | | | | | X | X | |
| 15 | 4440 | X | | X | | X | X | | | | | | |
| | | | | X | | | | | X | X | | | |
| | | | | X | | | | | | | | X | X |
| 16 | 4440 | X | | X | X | | X | | | | | | |
| | | | | X | | | | X | | X | | | |
| | | | | X | | | | | | | X | | X |
| 17 | 4440 | X | | X | | | X | | | | | | |
| | | | | X | | | | | | X | | | |
| | | | | X | | | | | | | | | X |

EP 3 908 822 B1

[Tableau 3B]

Tableau 3B

| Ref | L-T KIC/Kq | T-L KIC/Kq | S-L KIC/Kq | σ(ε) | σ(ε) (%) | Mean(ε) (%) |
|-----|-----------|-----------|-----------|------|----------|-------------|
| 9 | X | | | 0.94 | 2.74 | 2.04 |
| 10 | | X | | 0.88 | 2.91 | 2.16 |
| 11 | | | X | 1.17 | 3.63 | 2.85 |
| 12 | X | | | 1.23 | 3.76 | 2.96 |
| | | X | | | | |
| | | | X | | | |
| 13 | X | | | 1.58 | 4.74 | 3.70 |
| | | X | | | | |
| | | | X | | | |
| 14 | X | | | 1.29 | 3.95 | 3.11 |
| | | X | | | | |
| | | | X | | | |
| 15 | X | | | 1.34 | 4.08 | 3.20 |
| | | X | | | | |
| | | | X | | | |
| 16 | X | | | 1.29 | 3.93 | 3.08 |
| | | X | | | | |
| | | | X | | | |
| 17 | X | | | 1.36 | 4.16 | 3.27 |
| | | X | | | | |
| | | | X | | | |

[Tableau 4]

Tableau 4

| REF | Nb de données | Entrées | | | | Target | (σ) | | Mean (ε) |
|-----|---------------|---------|-----|-----|-----|--------|-----|-----|----------|
| | | Ep. | UTS (TL) | TYS (TL) | A% (TL) | V50 (m/s) | Valeur (m/s) | (%) | (%) |
| 18 | 448 | X | X | X | X | X | 0,28 | 0,039 | 0,05 |

**Revendications**

**1.** Procédé de contrôle d'une propriété de tolérance aux dommages d'une pièce réalisée en alliage d'aluminium, la pièce étant en forme de tôle ou de profilé extrudé, comportant les étapes suivantes :

a) mesurer au moins deux propriétés résultant d'essais de la traction de la pièce, dans les directions L(longitudinal) et/ou ST (travers court) et/ou LT (travers long), les propriétés étant choisies parmi :

- la limite d'élasticité ;
- et/ou la résistance à la traction ;
- et/ou l'allongement à la rupture ;

b) prendre en compte une épaisseur de la pièce ;
c) utiliser les propriétés mesurées lors de l'étape a) et l'épaisseur prise en compte lors de l'étape b) en tant que données d'entrée ($x_i$) d'un estimateur par réseaux de neurones ;
d) estimer, à l'aide de l'estimateur, la propriété représentative d'une tolérance aux dommages de la pièce ($\hat{z}, \hat{K}_{IC}$);
e) prendre en compte un seuil d'acceptation ($\tilde{z}, \tilde{K}_{IC}$) et un intervalle de confiance ($\varepsilon$), et comparer la propriété estimée ($\hat{z}, \hat{K}_{IC}$) lors de l'étape d) au seuil d'acceptation, en tenant compte de l'intervalle de confiance ;
f) en fonction de la comparaison :

- considérer que la pièce satisfait au contrôle ;
- ou considérer que la pièce ne satisfait pas au contrôle.

2. Procédé selon la revendication 1, dans lequel lorsque lors de l'étape f), la pièce ne satisfait pas au contrôle, le procédé comporte une étape g) de mesure de la propriété de tolérance aux dommages de la pièce à partir d'une éprouvette prélevée dans ladite pièce.

3. Procédé selon l'une quelconque des revendications précédentes, dans lequel l'étape c) comporte également une prise en compte d'une concentration d'au moins un élément d'alliage dans l'alliage d'aluminium.

4. Procédé selon l'une quelconque des revendications précédentes, dans lequel dans lequel l'étape a) comporte une mesure de l'allongement à la rupture et dans lequel l'étape c) comporte une prise en compte de l'allongement à la rupture ainsi mesurée.

5. Procédé selon la revendication précédente, dans lequel l'étape a) comporte une mesure de la limite d'élasticité et dans lequel l'étape c) comporte une prise en compte de la limite d'élasticité ainsi mesurée.

6. Procédé selon l'une quelconque des revendications précédentes, dans lequel l'étape c) comporte également une prise en compte d'une propriété de dureté.

7. Procédé selon l'une quelconque des revendications précédentes, dans lequel l'étape a) comporte uniquement une mesure de l'allongement à la rupture et de la limite d'élasticité, et dans lequel l'étape c) comporte une prise en compte de l'allongement à la rupture et de la limite d'élasticité ainsi mesurés.

8. Procédé selon l'une quelconque des revendications précédentes, dans lequel :

- lorsque l'étape d) comporte une estimation de la propriété représentative d'une tolérance aux dommages de la pièce selon les directions L-T (Longitudinal - Travers Long), l'étape c) comporte, au moins, une prise en compte de propriétés mesurées, lors de l'étape a), selon la direction L;
- lorsque l'étape d) comporte une estimation de la propriété représentative d'une tolérance aux dommages de la pièce selon les directions T-L, (Travers Long - Longitudinal), l'étape c) comporte, au moins, une prise en compte de propriétés mesurées, lors de l'étape a), selon la direction LT ;
- lorsque l'étape d) comporte une estimation de la propriété représentative d'une tolérance aux dommages de la pièce selon les directions S-L, (Travers Court - Longitudinal) l'étape c) comporte, au moins, une prise en compte de propriétés mesurées, lors de l'étape a), selon la direction ST.

9. Procédé selon l'une quelconque des revendications précédentes, dans lequel l'étape c) comporte une prise en compte de propriétés mesurées, lors de l'étape a), selon différentes directions.

10. Procédé selon la revendication 9, dans lequel l'étape c) comporte une prise en compte de trois propriétés mesurées, lors de l'étape a), et cela selon trois directions différentes.

11. Procédé selon l'une quelconque des revendications précédentes, dans lequel la propriété de tolérance aux dommages est un facteur d'intensité apparent ($K_{app}$) ou une valeur critique d'un facteur d'intensité ($K_{IC}$), également appelé ténacité, ou un facteur d'intensité de contrainte effectif pour une extension de fissure effective prédéterminée, par exemple 60 mm ($K_{R60}$).

**12.** Procédé selon la revendication 1, dans lequel la propriété de tolérance aux dommages est une vitesse limite de protection (V50).

**13.** Procédé selon l'une quelconque des revendications précédentes, dans lequel l'alliage d'aluminium est un alliage de la série 2XXX ou de la série 7XXX ou de la série 6XXX ou de la série 5XXX.

**14.** Procédé selon l'une quelconque des revendications précédentes, dans lequel l'étape c) est mise en oeuvre par une unité de traitement, par exemple un microprocesseur.

**Patentansprüche**

**1.** Verfahren zur Prüfung einer Schadenstoleranzeigenschaft eines Werkstücks aus einer Aluminiumlegierung, wobei das Werkstück die Form eines stranggepressten Blechs oder Profils aufweist, umfassend die folgenden Schritte:

a) Messen von mindestens zwei Eigenschaften, die sich aus der Zugfestigkeitsprüfung des Teils ergeben, in den Richtungen L (längs) und/oder ST (kurz quer) und/oder LT (lang quer), wobei die Eigenschaften ausgewählt werden aus:

- der Streckgrenze;
- und/oder der Zugfestigkeit;
- und/oder der Bruchdehnung;

b) Berücksichtigen der Dicke des Teils;
c) Verwenden der in Schritt a) gemessenen Eigenschaften und der in Schritt b) berücksichtigte Dicke als Eingabedaten (xi) für einen Schätzer eines neuronalen Netzes;
d) Schätzen der für eine Schadenstoleranz des Teils repräsentativen Eigenschaft ($\hat{z}$, $\hat{K}IC$) mit Hilfe des Schätzers;
e) Berücksichtigen einer Akzeptanzschwelle ($\tilde{z}$, $\check{K}_{IC}$) und eines Konfidenzintervalls ($\varepsilon$), und Vergleichen der in Schritt d) geschätzten Eigenschaft ($\tilde{z}$, $\check{K}_{IC}$) mit der Akzeptanzschwelle unter Berücksichtigung des Konfidenzintervalls;
f) in Abhängigkeit von dem Vergleich:

- davon ausgehen, dass das Teil die Prüfung besteht;
- oder davon ausgehen, dass das Teil die Prüfung nicht besteht.

**2.** Verfahren nach Anspruch 1, wobei, wenn das Teil während des Schritts f) die Prüfung nicht besteht, das Verfahren einen Schritt g) des Messens der Schadenstoleranzeigenschaft des Teils anhand eines dem Teil entnommenen Prüfstücks umfasst.

**3.** Verfahren nach einem der vorstehenden Ansprüche, wobei Schritt c) auch das Berücksichtigen einer Konzentration von mindestens einem Legierungselement in der Aluminiumlegierung umfasst.

**4.** Verfahren nach einem der vorstehenden Ansprüche, wobei Schritt a) das Messen der Bruchdehnung umfasst und wobei Schritt c) das Berücksichtigen der so gemessenen Bruchdehnung umfasst.

**5.** Verfahren nach dem vorstehenden Anspruch, wobei Schritt a) das Messen der Streckgrenze umfasst und wobei Schritt c) das Berücksichtigen der so gemessenen Streckgrenze umfasst.

**6.** Verfahren nach einem der vorstehenden Ansprüche, wobei Schritt c) auch das Berücksichtigen einer Härteeigenschaft umfasst.

**7.** Verfahren nach einem der vorstehenden Ansprüche, wobei Schritt a) nur das Messen der Bruchdehnung und der Streckgrenze umfasst, und wobei Schritt c) das Berücksichtigen der so gemessenen Bruchdehnung und Streckgrenze umfasst.

**8.** Verfahren nach einem der vorstehenden Ansprüche, wobei:

- wenn Schritt d) eine Schätzung der Eigenschaft umfasst, die für eine Schadenstoleranz des Teils entlang der Richtungen L-T (längs - lang quer) repräsentativ ist, umfasst Schritt c) mindestens das Berücksichtigen von Eigenschaften, die während Schritt a) entlang der Richtung L gemessen wurden;

- wenn Schritt d) eine Schätzung der Eigenschaft umfasst, die für eine Schadenstoleranz des Teils entlang der Richtungen T-L, (lang quer - längs) repräsentativ ist, umfasst Schritt c) mindestens das Berücksichtigen von Eigenschaften, die während Schritt a) entlang der Richtung LT gemessen wurden;

- wenn Schritt d) eine Schätzung der Eigenschaft umfasst, die für eine Schadenstoleranz des Teils entlang der Richtungen S-L (kurz quer - längs) repräsentativ ist, umfasst Schritt c) mindestens das Berücksichtigen von Eigenschaften, die während Schritt a) entlang der Richtung ST gemessen wurden.

9. Verfahren nach einem der vorstehenden Ansprüche, wobei Schritt c) das Berücksichtigen von in Schritt a) aus verschiedenen Richtungen gemessenen Eigenschaften umfasst.

10. Verfahren nach Anspruch 9, wobei in Schritt c) drei in Schritt a) gemessene Eigenschaften aus drei verschiedenen Richtungen berücksichtigt werden.

11. Verfahren nach einem der vorstehenden Ansprüche, wobei die Schadenstoleranzeigenschaft ein scheinbarer Intensitätsfaktor ($K_{app}$) oder ein kritischer Wert eines Intensitätsfaktors ($K_{IC}$), auch bekannt als Zähigkeit, oder ein effektiver Spannungsintensitätsfaktor für eine vorbestimmte effektive Rissausdehnung, z. B. 60 mm ($K_{R60}$), ist.

12. Verfahren nach Anspruch 1, wobei die Schadenstoleranzeigenschaft eine Schutzgrenzgeschwindigkeit (V50) ist.

13. Verfahren nach einem der vorstehenden Ansprüche, wobei die Aluminiumlegierung eine Legierung der 2XXX-Serie oder der 7XXX-Serie oder der 6XXX-Serie oder der SXXX-Serie ist.

14. Verfahren nach einem der vorstehenden Ansprüche, wobei der Schritt c) von einer Verarbeitungseinheit, z. B. einem Mikroprozessor, ausgeführt wird.

## Claims

1. Method for testing a damage-tolerant property in an aluminum alloy part, the part being in the shape of a metal sheet or extruded profile, with the following steps:

    a) measure at least two properties resulting from tensile testing of the part, in the L (longitudinal) and/or ST (short transverse) and/or LT (long transverse) directions, the properties being chosen from:

    - the yield strength;
    - and/or the tensile strength;
    - and/or the elongation at rupture;

    b) take into account the thickness of the part;
    c) use the properties measured during step a) along with the thickness taken into account during step b) as input parameters ($x_i$) for a neural network estimator;
    d) estimate, using the estimator, the property representative of a part's damage tolerance ($\hat{z}$, $\hat{K}_{IC}$);
    e) take into account an acceptance threshold ($\tilde{z}$, $\tilde{K}_{IC}$) and a confidence interval ($\varepsilon$), and compare the property estimated ($\hat{z}$, $\hat{K}_{IC}$) during step d) to the acceptance threshold, taking into account the confidence interval;
    f) based on the comparison:

    - consider that the part passes the test;
    - or consider that the part does not pass the test.

2. Method according to claim 1, in which when, during step f), the part fails the test, the method involves a step g) of measuring the part's damage tolerance property from a test specimen taken from that part.

3. Method according to any of the previous claims, in which step c) also involves taking into account the concentration of at least one alloy element in the aluminum alloy.

4. Method according to any of the previous claims, in which the step a) involves measuring the elongation at rupture and in which step c) involves taking into account the elongation at rupture thus measured.

5. Method according to the previous claim, in which step a) involves measuring the yield strength and in which step c) involves taking into account the yield strength thus measured.

6. Method according to any of the previous claims, in which step c) also involves consideration of a hardness property.

7. Method according to any of the previous claims, in which step a) consists only measuring the elongation at rupture and yield strength, and in which step c) involves consideration of the elongation at rupture and yield strength thus measured.

8. Method according to any of the previous claims in which:

   - when step d) comprises an estimation of the property representative of a damage tolerance of the part according to the L-T (Longitudinal - Long Transverse) directions, step c) comprises, at the least, consideration of the properties measured during step a), in direction L;
   - when step d) comprises an estimation of the property representative of a damage tolerance of the part according to the T-L (Long Transverse - Longitudinal) directions, step c) comprises, at the least, consideration of the properties measured during step a), in direction LT;
   - when step d) comprises an estimation of the property representative of a damage tolerance of the part according to the S-L (Short Transverse - Longitudinal) directions, step c) comprises, at the least, consideration of the properties measured during step a), in direction ST.

9. Method according to any of the previous claims in which step c) involves consideration of properties measured during step a), in several directions.

10. Method according to claim 9, in which step c) involves taking into account three properties measured in step a), in three different directions.

11. Method according to any of the previous claims, in which the damage tolerance property is an apparent intensity factor ($K_{app}$) or a critical value of an intensity factor ($K_{IC}$), also called fracture toughness, or an effective stress intensity factor for a predetermined effective crack extension, for example 60 mm ($K_{R60}$).

12. Method according to claim 1, in which the damage tolerance property is a ballistic limit velocity (V50).

13. Method according to any of the previous claims, in which the aluminum alloy is an alloy of the 2XXX series, or the 7XXX series, or the 6XXX series, or the 5XXX series.

14. Method according to any of the previous claims, in which step c) is implemented by a processing unit, such as a microprocessor.

[Fig 1]

**Fig. 1**

[Fig. 2A]

**Fig. 2A**

[Fig. 2B]

**Fig. 2B**

[Fig. 3A]

$\widehat{K}_{IC}$

$K_{IC}$

**Fig. 3A**

[Fig. 3B]

(MPavm)

**Fig. 3B**

[Fig 4A]

**Fig. 4A**

[Fig. 4B]

**Fig. 4B**

[Fig. 4C]

25

**Fig. 4C**

[Fig. 4D]

**Fig. 4D**

[Fig. 5A]

**Fig. 5A**

[Fig. 5B]

**Fig. 5B**

[Fig. 5C]

**Fig. 5C**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **PARTHEEPAN G.** Fracture toughness evaluation using miniature specimen and neural network. *Computational Materials Science,* 2008, vol. 44, 523-530

- **KANG JY.** Application of artificial neural network for predicting plain strain fracture toughness using tensile test results. *Fatigue Fact Engng Mater,* vol. 29, 321-329